# EUROPÄISCHE PATENTSCHRIFT

(11) **EP 2 804 568 B1**
(45) Veröffentlichungstag und Bekanntmachung des Hinweises auf die Patenterteilung: **13.01.2016**
(21) Anmeldenummer: 13700320.8
(22) Anmeldetag: 17.01.2013
(51) Int. Cl.: A61F 2/966

(54) **ANORDNUNG UMFASSEND EINEN DRAHT UND EIN MEDIZINISCHES IMPLANTAT**
ARRANGEMENT COMPRISING A WIRE AND A MEDICAL IMPLANT
SYSTÈME COMPRENANT UN FIL ET UN IMPLANT MÉDICAL

(30) Priorität: 17.01.2012 DE 102012100362
(43) Veröffentlichungstag der Anmeldung: 26.11.2014
(73) Patentinhaber: Acandis GmbH & Co. KG, 76327 Pfinztal (DE)
(72) Erfinder: BÜCHERT, Michael, 75015 Bretten (DE); BAIDINGER, Paul, 75179 Pforzheim (DE)
(74) Vertreter: Kilchert, Jochen
(86) Internationale Anmeldenummer: PCT/EP2013/050764
(87) Internationale Veröffentlichungsnummer: WO 2013/107783

(56) Entgegenhaltungen:
- EP-A1- 2 208 483
- WO-A1-2009/149457
- US-A1- 2007 255 385

## Beschreibung

Die Erfindung betrifft eine Anordnung, die einen Draht und ein komprimierbares, medizinisches Implantat umfasst, das mit dem Draht lösbar verbunden ist, wobei der Draht eine erste Hülse mit wenigstens einer Nase aufweist, die sich radial nach außen erstreckt, die mit dem Implantat zur Kraftübertragung in proximaler Richtung in Eingriff gebracht ist und die einen ersten Anschlag für das Implantat bildet, wobei eine zweite Hülse proximal zur ersten Hülse angeordnet ist, die einen zweiten Anschlag zur Kraftübertragung in distaler Richtung bildet, wobei ein Freiraum zur Aufnahme des Implantats vorgesehen ist, der distal durch die erste Hülse und proximal durch die zweite Hülse begrenzt ist.

Eine solche Anordnung ist bereits aus der US 6,120,522 bekannt. Dieses System beinhaltet einen Katheter und einen Stent, der mit dem dargestellten Systemaufbau an seinen Einsatzort, z.B. an den Ort der Stenose, transportiert wird. Um dieses Ziel zu erreichen, umfasst das System einen Ring, der mit Hilfe eines Drahtes an eine Stoßleiste andockt, um durch Kraftübertragung den Stent aus dem System herauszuschieben. Der Stent wird vorher mit einem Stent-Halter versehen, der sich vom Stent erst dann löst, wenn sich das System an dem Einsatzort befindet. Des Weiteren umfasst das System Marker, um die Position des Stents mit Hilfe von Röntgenaufnahmen festzustellen.

Das beschriebene System hat den Nachteil, dass für die Platzierung der Bauteile im Katheter verhältnismäßig viel Raum benötigt wird.

Ähnliche Anordnungen sind aus EP 2 208 483 A1, WO 2009/149457 A1 und US 2007/0255385 A1 bekannt. Die bekannten Anordnungen nutzen einen Transportdraht zur Zuführung eines Stents durch einen Katheter an den Behandlungort, wobei der Transportdraht Eingriffselemente aufweist, die direkt auf Stege des Stents einwirken. Ist der Behandlungsort nur über stark gewundene Blutgefäße erreichbar, so ist eine hohe Biegeflexibilität des Drahtes und des Stents erwünscht. Um dies zu erreichen, können der Draht und die Stege des Stents vergleichweise dünn ausgeführt sein. Daraus ergeben sich Schwierigkeiten beim Schieben des Stents durch den Katheter, da der dünne Draht und die dünnen Stege den Vorschubkräften nicht sicher standhalten. Eine Erhöhung der Drahtstärke oder Stegdicke führt hingegen zu einem erhöhten Raumbedarf innerhalb des Katheters.

Aufgabe der Erfindung ist es daher, eine Anordnung zu schaffen, bei der der Raumbedarf für den Einbau der Einzelelemente verringert ist, wobei das Implantat im Gefäß präzise positionierbar ist.

Diese Aufgabe wird erfindungsgemäß durch die Anordnung nach Anspruch 1 gelöst. Insbesondere wird die Aufgabe durch eine Anordnung gelöst, die einen Draht und ein komprimierbares, medizinisches Implantat umfasst, das mit dem Draht lösbar verbunden ist. Dabei weist der Draht eine erste Hülse mit wenigstens einer Nase auf, die sich radial nach außen erstreckt. Die Nase ist mit dem Implantat zur Kraftübertragung in proximaler Richtung in Eingriff gebracht und bildet einen ersten Anschlag für das Implantat. Der Draht weist eine zweite Hülse auf, die proximal zur ersten Hülse angeordnet ist und einen zweiten Anschlag zur Kraftübertragung in distaler Richtung bildet. Außerdem ist ein Freiraum zur Aufnahme des Implantats vorgesehen, der distal durch die erste Hülse und proximal durch die zweite Hülse begrenzt ist. Das Implantat weist dabei wenigstens eine Öse mit Röntgenmarkern auf, die im komprimierten Zustand des Implantats im Freiraum angeordnet sind. Durch diese Anordnung erhält die Öse eine Doppelfunktion. Zum Einen wird ein geringer Einbauraum benötigt, da die Öse im Freiraum befindlich ist und der verfügbare Platz damit optimal ausgenutzt wird. Zum Anderen bleibt die Funktion der Positionsbestimmung des Implantats durch die Röntgenmarker erhalten.

In vorteilhafter Ausgestaltung der Erfindung ist vorgesehen, dass das proximale Ende der Öse eine abgerundete Form aufweist, da durch diese Formgebung eine verbesserte Kraftübertragung erreicht wird. Die zweite Hülse dient für die Öse als Anschlag zur Kraftübertragung in distaler Richtung.

In weiterer vorteilhafter Ausgestaltung der Erfindung ist vorgesehen, dass die Form der Nase der Form des Zwischenraums der Stege des Implantats angepasst ist. Dadurch wird eine sichere Verbindung zwischen der Nase der ersten Hülse und den Stegen erzeugt. Durch diesen konstruktiven Vorteil wird eine optimale Kraftübertragung erreicht.

Vorzugsweise ist vorgesehen, dass die Endzellen mit Markern länger als die markerlosen Endzellen ausgebildet sind. Dadurch wird die kompakte Anordnung des Implantats im Zufuhrsystem weiter verbessert, da das Implantat im Bereich der Ösen auf einen kleinen Durchmesser komprimiert werden kann.

Bei einer vorteilhaften Ausgestaltung ist vorgesehen, dass der Draht am distalen Ende einen Abschnitt aufweist, der in distaler Richtung über die erste Hülse zur Stabilisierung des Implantats axial vorsteht. Damit wird erreicht, dass der Transport des Drahtes durch den Katheter leicht und sicher erfolgt, und das Implantat geführt ist.

In einem weiteren Ausführungsbeispiel ist vorgesehen, dass die erste Hülse mindestens 2 Nasen aufweist, insbesondere mindestens 3 Nasen, insbesondere mindestens 4 Nasen, insbesondere mindestens 5 Nasen, insbesondere mindestens 6 Nasen, insbesondere mindestens 7 Nasen, insbesondere mindestens 8 Nasen, insbesondere mindestens 9 Nasen, insbesondere mindestens 10 Nasen und insbesondere mindestens 12 Nasen. Damit weist das Implantat eine stabile Lage auf und kann nicht verrutschen.

In weiterer vorteilhafter Ausgestaltung ist vorgesehen, dass das Implantat eine der Anzahl der Nasen entsprechende Anzahl an Ösen, insbesondere drei Ösen aufweist, die in einem Winkel von 120° angeordnet sind. In jeder Öse ist ein Röntgenmarker platziert. Mit dieser Anordnung der Ösen wird die Positionierbarkeit des Implantats verbessert, da lageunabhängig praktisch der gesamte Umfang des Implantats sichtbar ist.

Vorzugsweise ist vorgesehen, dass eine zweite Hülse eine der Anzahl der Nasen der ersten Hülse entsprechende Anzahl an Nasen aufweist, insbesondere wenigstens eine Nase, insbesondere mindestens 2 Nasen, insbesondere mindestens 3 Nasen, insbesondere mindestens 4 Nasen, insbesondere mindestens 5 Nasen, insbesondere mindestens 6 Nasen, insbesondere mindestens 7 Nasen, insbesondere mindestens 8 Nasen, insbesondere mindestens 9 Nasen, insbesondere mindestens 10 Nasen und insbesondere mindestens 12 Nasen.

Der Vorteil dieser Ausführungsform besteht darin, dass die Nasen der ersten Hülse und die Nasen der zweiten Hülse fluchtend angeordnet werden können, wenn die Anzahl der Nasen der ersten Hülse der Anzahl der Nasen der zweiten Hülse entspricht. Durch die fluchtende Anordnung der Nasen fluchten automatisch auch die Zwischenräume zwischen den Nasen, die jeweils in Umfangsrichtung ausgebildet ist. Die erste und zweite Hülse weisen vorzugsweise im Bereich der Zwischenräume jeweils den gleichen mittleren Durchmesser auf. Allgemein weisen die Hülsen einen Innendurchmesser, einen mittleren Durchmesser und einen Außendurchmesser auf. Der Innendurchmesser bezieht sich auf den Umfang der Bohrung der Hülsen, die die Durchgangsöffnung für die Seele darstellen. Der mittlere Durchmesser bezieht sich auf den Umfang der Hülse im Bereich der Zwischenräume der Nasen. Der Außendurchmesser bezieht sich auf den Umfang der Hülse, der durch die Nasenrücken, d.h. durch die radialen Außenflächen der Nasen gebildet ist. Die fluchtende Anordnung zusammen mit den übereinstimmenden mittleren Durchmesser hat den fertigungstechnischen Vorteil, dass zum Befestigen der ersten und zweiten Hülse auf der Seele des Drahtes mittels Punktschweißung dieselben Laserparameter verwendet werden können. Der weitere Vorteil der fluchtenden Anordnung besteht darin, dass die im Freiraum angeordneten Ösen zwangsläufig auf der Höhe der Nasen der proximal angeordneten zweiten Hülse liegen. Damit ist die Anschlagfläche in radialer Richtung maximal (verglichen mit der kleineren Anschlagsflächen im Bereich der Zwischenräume zwischen den Nasen).

Bei einem weiteren Ausführungsbeispiel ist vorgesehen, dass die Seele am distalen Ende, insbesondere im Bereich des vorstehenden Abschnittes, einen geringeren Durchmesser aufweist als am proximalen Ende, insbesondere in einem Bereich proximal zum vorstehenden Abschnitt. Dadurch wird die Flexibilität des distalen Endes der Seele im Vergleich zur Flexibilität des proximalen Teils der Seele erhöht. Dies hat den Vorteil, dass die Katheterspitze besser in kleine und stark gewundene Gefäße eingeführt werden kann.

Zweckmäßigerweise weist der Draht wenigstens eine lose Hülse auf. Die lose Hülse dient als Röntgenmarker und ist proximal zur zweiten Hülse angeordnet. Die Lage der losen Hülse zeigt dem Operateur an, ob das Implantat nur soweit aus dem Katheter entlassen ist, dass dieses wieder eingezogen werden kann oder ob dieses bereits vollständig entlassen ist und nicht wieder eingezogen werden kann (Point of no Return).

In einer weiteren Ausgestaltung ist vorgesehen, dass die erste Hülse fest, insbesondere stoffschlüssig, insbesondere mittels Schweißen, Kleben oder Löten, oder durch plastische Verformung, insbesondere mittels Crimpen, mit der Seele verbunden ist.

In einem weiteren vorteilhaften Ausführungsbeispiel ist vorgesehen, dass die erste Hülse um die Seele rotierbar gelagert ist. Durch diese Ausgestaltung ist die Anordnung flexibler gestaltet und ist somit leichter durch stark gekrümmten Hohlkörper führbar. Dies resultiert daraus, dass durch eine Krümmung der Anordnung auch das Implantat gekrümmt wird. Mit der Krümmung des Implantats geht aber auch eine Rotation desselben einher, welche sich auf die erste Hülse auswirkt. Indem die erste Hülse um die Seele rotierbar gelagert ist, können somit mechanische Spannungen zwischen Implantat und Draht reduziert oder verhindert werden.

In weiterer vorteilhafter Ausgestaltung ist vorgesehen, dass die erste Hülse auf ihrer distalen Seite durch das Coil und auf ihrer proximalen Seite durch einen Ring axial fixiert ist. Dadurch ist die erste Hülse mit einfachen Mitteln an einer vordefinierten Stelle axial gehalten.

In weiterer vorteilhafter Ausgestaltung ist vorgesehen, dass der Ring fest, insbesondere stoffschlüssig, insbesondere mittels Schweißen, Kleben oder Löten, oder durch plastische Verformung, insbesondere mittels Crimpen, mit der Seele verbunden ist.

In weiterer vorteilhafter Ausgestaltung ist vorgesehen, dass der Ring rotationssymmetrisch ausgestaltet ist. In weiterer vorteilhafter Ausgestaltung ist vorgesehen, dass der Ring einen Querschnitt aufweist, der bspw. kreisförmig, oval, dreieckig, quadratisch, rechteckig oder vieleckig gebildet ist.

In weiterer vorteilhafter Ausgestaltung ist vorgesehen, dass die radiale Ausdehnung des Rings kleiner als die radiale Ausdehnung der ersten Hülse ist. Mit radialer Ausdehnung ist der Durchmesser des Rings, d.h. die Ausdehnung senkrecht zur Seele, gemeint. Mit anderen Worten ist der Außendurchmesser der ersten Hülse größer, als der Außendurchmesser des Rings. Insbesondere ist die radiale Ausdehnung des Rings kleiner als der Außendurchmesser der Nase der ersten Hülse. Insbesondere ist die radiale Ausdehnung des Rings kleiner als die Wurzeldurchmesser der Nase der ersten Hülse. Dadurch ist ein Eingriff des Implantats mit der Nase der ersten Hülse zur Kraftübertragung weiterhin möglich und die Nase der ersten Hülse bildet weiterhin einen ersten Anschlag für das Implantat. Die radiale Ausdehnung des Rings ist ausreichend groß ausgebildet, so dass eine erste, zur ersten Hülse gerichtete Stirnfläche des Rings und die Stirnfläche der ersten Hülse sich überlappen. Dadurch bildet der Ring einen Anschlag für die erste Hülse, wobei der Ring eine axiale Verschiebung der ersten Hülse in proximaler Richtung verhindert.

Alternativ kann vorgesehen sein, dass die axiale Ausdehnung des Rings der axialen Ausdehnung des Freiraums entspricht. Die axiale Ausdehnung des Rings kann kürzer sein als die axiale Ausdehnung des Freiraums. Dabei kann vorgesehen sein, dass die axiale Ausdehnung des Rings derart gestaltet ist, dass sich der Ring nur in einen Teilbereich des Freiraums zwischen Öse des Implantats und erster Hülse erstreckt. Dabei übergreift die Öse den ring und erstreckt sich in den proximal vom Ring angeordneten Teilbereich des Freiraums, d.h. in den ringförmigen Bereich des Freiraums. Der Abschnitt der Öse oder der mit der Öse verbundenen Stege, der den Ring übergreift, kann auf dem Ring aufliegen oder von diesem beabstandet sein. Mit axialer Ausdehnung ist die Länge des Rings entlang seiner Längs- oder Rotationsachse, d.h. entlang der Seele, gemeint.

Dabei ist die radiale Ausdehnung des Rings so groß, dass einerseits in radialer Richtung ein ausreichend großer Bereich des Freiraums bestehen bleibt, um die Öse aufzunehmen. Der radiale Außendurchmesser des Rings ist deshalb nur geringfügig größer als der radiale Außendurchmesser Seele. Andererseits ist der radiale Außendurchmesser des Rings so groß, dass die Stirnfläche des Rings und der ersten Hülse zur Bildung des Anschlags überlappen.

Zusammengefasst besteht die Funktion des Rings darin eine axiale Verschiebung der ersten Hülse in proximaler Richtung, also in den Freiraum hinein, zu verhindern.

Die Erfindung wird im Folgenden anhand schematischer Zeichnungen beispielsweise und mit weiteren Einzelheiten erläutert. In diesen zeigen:
- Fig.1: eine perspektivische Darstellung der Anordnung mit einem Draht;
- Fig.2: eine weitere perspektivische Darstellung der Anordnung mit einem Draht;
- Fig.3: eine perspektivische Darstellung der Anordnung mit einem Draht und einem medizinischen Implantat; und
- Fig.4: eine perspektivische Darstellung einer weiteren Anordnung mit einem Draht

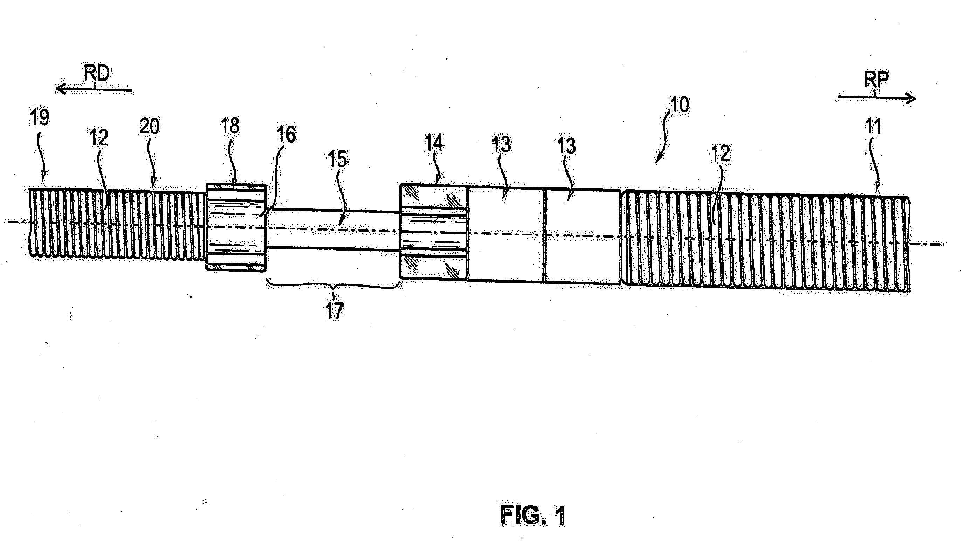

Figur 1 zeigt eine perspektivische Darstellung einer Anordnung, die einen Draht 10 aufweist. Der Draht 10 wird im Gebrauch zum Transport eines komprimierten Implantats, beispielsweise eines Stents, in einem Zufuhrsystem, insbesondere in einem Katheter, verwendet (Transportdraht). Hierbei wird ein Katheter vorzugsweise der Größe 5 F verwendet, insbesondere 3 F, insbesondere 2,5 F, insbesondere 2 F, insbesondere 1 F (1 F = 0,33 mm). Der Draht 10 weist vom proximalen Ende 11 bis zum distalen Ende 19 eine Seele 15 auf, die vorzugsweise aus Nitinol oder Federstahl hergestellt ist und an ihrem distalen Ende 19 einen geringeren Durchmesser aufweist als am proximalen Ende 11. Dadurch ist der Draht am distalen Ende 11 flexibel. Zusammen mit den Coils 12, die sich sowohl am proximalen Ende 11 als auch am distalen Ende 19 befinden, erhält der Draht eine stabile Form und ein Verkanten im Katheter wird vermieden. Einzelne oder mehrere Drahtkomponenten, wie die Coils 12 sind hydrophil, hydrophob, mit PTFE, mit Parylene, biokompatibel oder anders beschichtbar.

Der Durchmesser der Seele 15 ist kleiner als 1,25 mm, insbesondere kleiner als 1,0 mm, insbesondere kleiner als 0,75 mm, insbesondere kleiner als 0,6 mm, insbesondere kleiner als 0,5 mm, insbesondere kleiner als 0,4 mm, insbesondere kleiner als 0,3 mm, insbesondere kleiner als 0,25 mm. Die Untergrenze kann 0,24 mm betragen.

Des Weiteren weist der Draht zwei Hülsen auf, die mit Hilfe von Laserschneiden oder Ätztechnik oder durch andere Prozesse hergestellt werden. Die beiden Hülsen bestehen bevorzugt aus Nitinol oder aus Edelmetall wie Platin, Hartmetall oder Kunststoff. Die Hülsen weisen eine Dicke (axiale Tiefe) auf kleiner als 0,75 mm, insbesondere kleiner als 0,5 mm, insbesondere kleiner als 0,4 mm, insbesondere kleiner als 0,3 mm, insbesondere kleiner als 0,25 mm, insbesondere kleiner als 0,2 mm, insbesondere kleiner als 0,15 mm, insbesondere kleiner als 0,1 mm. Die Untergrenze kann 0,05 mm betragen.

Zusätzlich umfassen die Hülsen drei Nasen 18. Generell können die Hülsen jeweils wenigstens eine Nase 18, insbesondere mindestens 2 Nasen, insbesondere mindestens 3 Nasen, insbesondere mindestens 4 Nasen, insbesondere mindestens 5 Nasen, insbesondere mindestens 6 Nasen, insbesondere mindestens 7 Nasen, insbesondere mindestens 8 Nasen, insbesondere mindestens 9 Nasen, insbesondere mindestens 10 Nasen und insbesondere mindestens 12 Nasen aufweisen, die sich radial nach außen und parallel zur Mittelachse der jeweiligen Hülse erstrecken. Die Breite der Nase 18 der ersten Hülse ist kleiner als 0,3 mm, insbesondere kleiner als 0,25 mm, insbesondere kleiner als 0,2 mm, insbesondere kleiner als 0,15 mm, insbesondere kleiner als 0,125 mm, insbesondere kleiner als 0,1 mm, insbesondere kleiner als 0,09 mm, insbesondere kleiner als 0,08 mm, insbesondere kleiner als 0,07 mm, insbesondere kleiner als 0,06 mm, insbesondere kleiner als 0,05 mm. Die Untergrenze kann 0,04 mm betragen. Die Nasen 18 der zweiten Hülse 14 sind breiter als die Nasen der ersten Hülse 16.

Die Hülsen weisen auf Grund der Nasen einen Außendurchmesser kleiner als 1,0 mm, insbesondere kleiner als 0,8 mm, insbesondere kleiner als 0,75 mm, insbesondere kleiner als 0,7 mm, insbesondere kleiner als 0,6 mm, insbesondere kleiner als 0,5 mm, insbesondere kleiner als 0,45 mm, insbesondere kleiner als 0,4 mm, insbesondere kleiner als 0,35 mm, insbesondere kleiner als 0,3 mm, insbesondere kleiner als 0,25 mm auf. Die Untergrenze kann 0,2 mm betragen. Der Innendurchmesser ist kleiner als 0,5 mm, insbesondere kleiner als 0,4 mm, insbesondere kleiner als 0,3 mm, insbesondere kleiner als 0,25 mm, insbesondere kleiner als 0,2 mm, insbesondere kleiner als 0,15 mm, insbesondere kleiner als 0,1 mm. Die Untergrenze kann 0,05 mm betragen.

Die Nase 18 der ersten Hülse 16, die auf der Seele 15 des Drahtes 10 angeordnet ist, ist mit dem Implantat 21 zur Kraftübertragung in proximaler Richtung RP in Eingriff gebracht und bildet einen ersten Anschlag für das Implantat 21. Die zweite Hülse 14, die proximal zur ersten Hülse 16 angeordnet ist, bildet einen zweiten Anschlag zur Kraftübertragung in distaler Richtung RD. Außerdem ist ein Freiraum 17 zur Aufnahme des Implantats 21 vorgesehen, der distal durch die erste Hülse 16 und proximal durch die zweite Hülse 14 begrenzt ist.

Beide Hülsen umfassen Nasen 18, die so auf der Seele 15 im Freiraum 17 angeordnet sind, dass die Nasen 18 der ersten Hülse 16 und die Nasen 18 der zweiten Hülse 14 fluchten. Außerdem fluchten auch die Zwischenräume der Nasen 18, die den mittleren Durchmesser der Hülsen bilden. Dies hat den Vorteil, dass dadurch die Hülsen vorzugsweise durch Schweißen mit der Seele 15 verbunden werden können und die beiden Hülsen mit gleichen Schweißparametern befestigt werden können. Die beiden Hülsen können auch durch Löten oder Kleben auf der Seele 15 befestigt werden.

Auf der proximalen Seite der zweiten Hülse 14 ist wenigstens eine lose Hülse 13 auf der Seele 15 derart befestigt, dass die lose Hülse 13 an der zweiten Hülse 14 anliegt. Vorzugsweise werden 2 lose Hülsen auf der Seele 15 angeordnet, da sie zum einen dem Draht 10 eine flexible Form geben. Zum Anderen wird sichergestellt, dass die beiden losen Hülsen 13 bei den Röntgenaufnahmen sichtbar sind und der "Point of no Return" gesetzt ist; dies bedeutet, dass der operierende Arzt genau den Punkt sieht, bei dem das Implantat sich vom Draht löst.

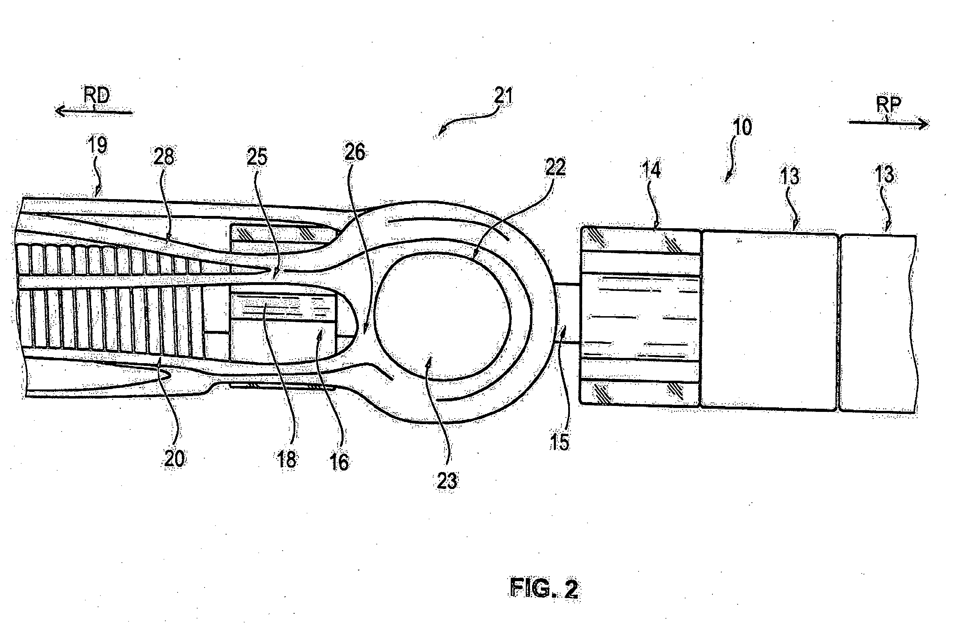

Fig. 2 zeigt eine weitere perspektivische Darstellung der Anordnung, die einen Draht aufweist. Der Aufbau entspricht dem Aufbau aus der Fig.1, wobei die Nasen 18 gut zu erkennen sind.

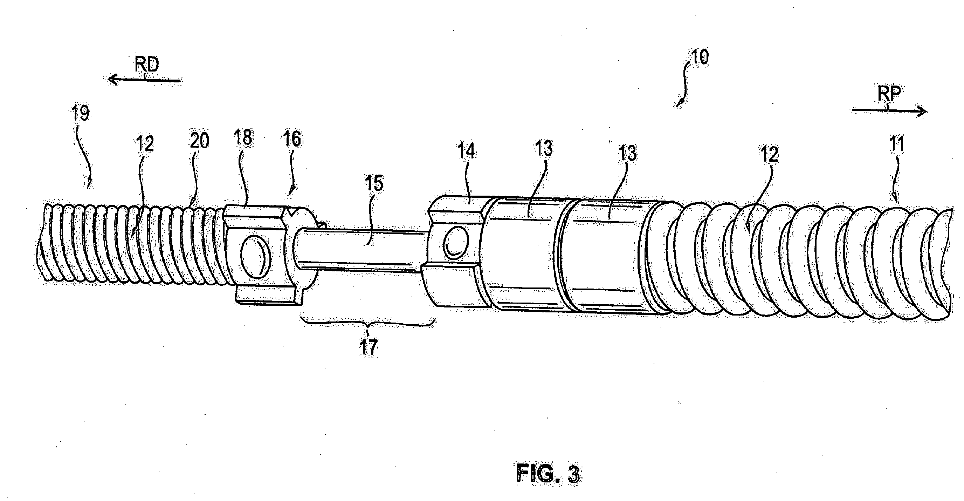

Fig.3 zeigt eine perspektivische Darstellung einer Anordnung, die einen Draht 10 und ein Implantat 21 aufweist. Am proximalen Ende 11 der Anordnung befinden sich die beiden losen Hülsen 13 und die zweite Hülse 14. In distaler Richtung RD ist auf der Seele 15 die erste Hülse 16 angebracht, wobei sich zwischen der ersten Hülse 16 und der zweiten Hülse 14 ein Freiraum 17 befindet. Die erste Hülse 16 und die zweite Hülse 16 weisen jeweils drei Nasen 18 auf, die unter einem Winkel von je 120° auf dem Umfang verteilt angeordnet sind. Zu den weiteren Einzelheiten wird auf die Ausführungen zu Fig. 1, 2 verwiesen.

Das Implantat 21 umfasst vorzugsweise drei Ösen 22, in denen jeweils ein Röntgenmarker 23 angeordnet ist. Die Ösen bilden das proximale Ende des Implantats 21. Die Ösen 22 befinden sich im Freiraum 17. Dies bedeutet, dass die Ösen 22 in dem Freiraum 17 zumindest teilweise versenkt sind, sodass die Ösen 22 mit der ersten und zweiten Hülse (16,14) zumindest teilweise überlappen. Am proximalen Ende weist die Öse 22 eine abgerundete Form 24 auf, die einen Anschlag mit der zweiten Hülse 14 bildet. Das Implantat 21 umfasst am distalen Ende Stege 25, die auf den Nasen 18 der ersten Hülse 16 anliegen.

Die Form des Zwischenraums 26 der Stege 25 ist der Form der Nase 18 angepasst. Die Nase 18 bildet einen Anschlag mit den Stegen 25, sodass die Kraftübertragung in proximaler Richtung RP erfolgt. Am distalen Ende 19 befindet sich der Fortführungsdraht 20. Um einen kompakten Bauraum zu erhalten sind die Endzellen mit Markern 27 länger ausgebildet als die markerlosen Endzellen 28. Dadurch wird ein besonders kleiner Crimpdurchmesser des Implantats erreicht.

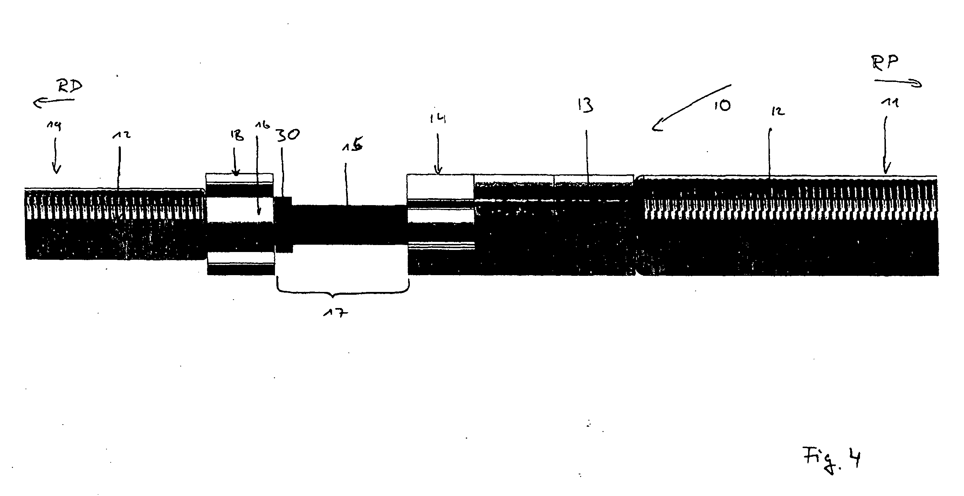

Fig. 4 zeigt eine perspektivische Darstellung einer Anordnung, die der Anordnung gemäß Fig. 1 im Wesentlichen entspricht. Im Gegensatz zur Anordnung gemäß Fig. 1 ist in der Anordnung gemäß Fig. 4 zur axialen Fixierung der ersten Hülse 16 auf der distalen Seite der ersten Hülse 16 das Coil 12 und auf der proximalen Seite der ersten Hülse 16 ein Ring 30 vorgesehen. Die erste Hülse 16 ist lose auf die Seele 15 geschoben und nicht mit ihr verbunden, so dass diese rotierbar ist. Der Ring 30 ist bspw. mittels Schweißen, Löten, Kleben oder Crimpen mit der Seele 15 fest verbunden und verhindert so eine axiale Verschiebung der ersten Hülse 16 in proximaler Richtung RP. Dies hat den Vorteil, dass das Implantat 21 zusammen mit der ersten Hülse 16 um die eigen Achse bzw. die Seele 15 rotieren kann, was die Führung des Katheters durch stark gekrümmte Blutgefäße erleichtert und mechanische Spannungen zwischen Implantat 21 und Draht 10 verhindert.

Die radiale und axiale Ausdehnung des Rings 30 ist derart gestaltet, dass sich der Ring 30 in den Raum zwischen der Öse 22 des Implantats 21 und der ersten Hülse 16 einfügt. Dies hat den Vorteil, dass der Freiraum 17 für das Implantat 21 bzw. für die Öse 22 nicht durch den Ring 30 blockiert wird. Gleichzeitig verhindert der Ring 30 eine axiale Verschiebung der ersten Hülse 16 in proximaler Richtung.

### Bezugszeichenliste

- 10: Draht
- 11: proximales Ende
- 12: Coil
- 14: zweite Hülse
- 15: Seele
- 16: erste Hülse
- 17: Freiraum
- 18: Nasen
- 19: distales Ende
- 20: Abschnitte
- 21: Implantat
- 22: Öse
- 23: Röntgenmarker
- 24: abgerundete Form
- 25: Stege
- 26: Zwischenraum
- 27: Marker
- 28: Endzellen

## Patentansprüche

1. Anordnung umfassend einen Draht (10) und ein komprimierbares, medizinisches Implantat (21), das mit dem Draht (10) lösbar verbunden ist, wobei der Draht (10) eine erste Hülse (16) mit wenigstens einer Nase (18) aufweist, die sich radial nach außen erstreckt, die mit dem Implantat (21) zur Kraftübertragung in proximaler Richtung (RP) in Eingriff gebracht ist und die einen ersten Anschlag für das Implantat bildet, wobei eine zweite Hülse (14) proximal zur ersten Hülse (16) angeordnet ist, die einen zweiten Anschlag zur Kraftübertragung in distaler Richtung (RD) bildet, wobei ein Freiraum (17) zur Aufnahme des Implantats (21) vorgesehen ist, der distal durch die erste Hülse (16) und proximal durch die zweite Hülse (14) begrenzt ist,
**dadurch gekennzeichnet, dass**
der Draht (10) eine Seele (15) aufweist, die am distalen Ende einen geringeren Durchmesser als in einem proximalen Bereich aufweist, und das Implantat (21) wenigstens eine Öse (22) mit Röntgenmarkern (23) aufweist, die im komprimierten Zustand des Implantats (21) im Freiraum (17) angeordnet ist.

2. Anordnung nach Anspruch 1,
**dadurch gekennzeichnet, dass**
das proximale Ende der Öse (22) eine abgerundete Form (24) aufweist und als Anschlag an die zweite Hülse (14) zur Kraftübertragung in distaler Richtung RD dient.

3. Anordnung nach Anspruch 1 oder 2,
**dadurch gekennzeichnet, dass**
die Form der Nase (18) der Form des Zwischenraums (26) der Stege (25) des Implantats angepasst ist.

4. Anordnung nach einem der vorhergehenden Ansprüche,
**dadurch gekennzeichnet, dass**
die Endzellen mit Markern (27) länger als die markerlosen Endzellen (28) ausgebildet sind.

5. Anordnung nach einem der vorhergehenden Ansprüche,
**dadurch gekennzeichnet, dass**
der Draht (10) am distalen Ende (RD) einen Abschnitt (20) aufweist, der in distaler Richtung (RD) über die erste Hülse (16) zur Stabilisierung des Implantats (21) axial vorsteht.

6. Anordnung nach einem der vorhergehenden Ansprüche,
**dadurch gekennzeichnet, dass**
die erste Hülse (16) mindestens 2 Nasen (18), insbesondere mindestens 3 Nasen (18), insbesondere mindestens 4 Nasen (18), insbesondere mindestens 5 Nasen (18), insbesondere mindestens 6 Nasen (18), insbesondere mindestens 7 Nasen (18), insbesondere mindestens 8 Nasen (18), insbesondere mindestens 9 Nasen (18), insbesondere mindestens 10 Nasen (18) und insbesondere mindestens 12 Nasen (18) umfasst.

7. Anordnung nach einem der vorhergehenden Ansprüche,
**dadurch gekennzeichnet, dass**
das Implantat (21) eine der Anzahl der Nasen (18) entsprechende Anzahl an Ösen, insbesondere drei Ösen (22) aufweist, die in einem Winkel von 120° angeordnet sind.

8. Anordnung nach einem der vorhergehenden Ansprüche,
**dadurch gekennzeichnet, dass**
die zweite Hülse (14) eine der Anzahl der Nasen (18) der ersten Hülse (16) entsprechende Anzahl an Nasen (18) aufweist, insbesondere wenigstens eine Nase (18), insbesondere mindestens 2 Nasen (18), insbesondere mindestens 3 Nasen (18), insbesondere mindestens 4 Nasen (18), insbesondere mindestens 5 Nasen (18), insbesondere mindestens 6 Nasen (18), insbesondere mindestens 7 Nasen (18), insbesondere mindestens 8 Nasen (18), insbesondere mindestens 9 Nasen (18), insbesondere mindestens 10 Nasen (18) und insbesondere mindestens 12 Nasen (18) umfasst.

9. Anordnung nach einem der vorhergehenden Ansprüche,
**dadurch gekennzeichnet, dass**
der Draht wenigstens eine lose Hülse aufweist, die als Röntgenmarker proximal zur zweiten Hülse (14) angeordnet ist.

10. Anordnung nach einem der vorhergehenden Ansprüche,
**dadurch gekennzeichnet, dass**
die erste Hülse (16) fest, insbesondere stoffschlüssig, insbesondere mittels Schweißen, Kleben oder Löten, oder durch plastische Verformung, insbesondere mittels Crimpen, mit der Seele (15) verbunden ist.

11. Anordnung nach einem der Ansprüchen 1 bis 9,
**dadurch gekennzeichnet, dass**
die erste Hülse (16) um die Seele (15) rotierbar gelagert ist.

12. Anordnung nach Anspruch 11,
**dadurch gekennzeichnet, dass**
die erste Hülse (16) auf ihrer distalen Seite durch ein Coil (12) und auf ihrer proximalen Seite durch einen Ring (30) axial fixiert ist.

13. Anordnung nach einem der Ansprüche 11 oder 12,
**dadurch gekennzeichnet, dass**
der Ring (30) fest, insbesondere stoffschlüssig, insbesondere mittels Schweißen, Kleben oder Löten, oder durch plastische Verformung, insbesondere mittels Crimpen, mit der Seele (15) verbunden ist.

14. Anordnung nach einem der Ansprüche 11, 12 oder 13,
**dadurch gekennzeichnet, dass**
die radiale Ausdehnung des Rings (30) kleiner ist als die radiale Ausdehnung der ersten Hülse (16).

## Claims

1. Arrangement comprising a wire (10) and a compressible medical implant (21), which is detachably connected to the wire (10), wherein the wire (10) has a first sleeve (16) with at least one lug (18) which extends radially outwards, is brought into engagement with the implant (21) in order to transmit force in the proximal direction (RP) and forms a first stop for the implant, wherein a second sleeve (14) is arranged proximally to the first sleeve (16) which forms a second stop for force transmission in the distal direction (RD), wherein a free space (17) is provided for receiving the implant (21) and is distally delimited by the first sleeve (16) and proximally by the second sleeve (14),
**characterised in that**
the wire (10) has a core (15) which at the distal end has a smaller diameter than in the proximal region, and **in that** the implant (21) has at least one eyelet (22) with X-ray markers (23) which in the compressed state of the implant (21) is arranged in the free space (17).

2. Arrangement according to claim 1,
**characterised in that**
the proximal end of the eyelet (22) has a rounded shape (24) and acts as a stop on the second sleeve (14) for force transmission in the distal direction RD.

3. Arrangement according to claim 1 or 2,
**characterised in that**
the shape of the lug (18) is adapted to the intermediate spacing (26) of the webs (25) of the implant.

4. Arrangement according to any one of the preceding claims,
**characterised in that**
the end cells with markers (27) are longer than the markerless end cells (28).

5. Arrangement according to any one of the preceding claims,
**characterised in that**
at the distal end (RD) the wire (10) has a section (20) which in the distal direction (RD) protrudes beyond the first sleeve (16) in order to stabilise the implant (21).

6. Arrangement according to any one of the preceding claims,
**characterised in that**
the first sleeve (16) comprises at least 2 lugs (18), more particularly at least 3 lugs (18), more particularly at least 4 lugs (18), more particularly at least 5 lugs (18), more particularly at least 6 lugs (18), more particularly at least 7 lugs (18), more particularly at least 8 lugs (18), more particularly at least 9 lugs (18), more particularly at least 10 lugs (18), and more particularly at least 12 lugs (18).

7. Arrangement according to at least one of the preceding claims,
**characterised in that**
the implant (21) has a number of eyelets corresponding to the number of lugs (18), more particularly three eyelets (22), which are arranged at an angle of 120°.

8. Arrangement according to at least one of the preceding claims,
**characterised in that**
the second sleeve (14) has a number of lugs (18) corresponding with the number of lugs (18) of the first sleeve (16), more particularly at least one lug (18), more particularly at least 2 lugs (18), more particularly at least 3 lugs (18), more particularly at least 4 lugs (18), more particularly at least 5 lugs (18), more particularly at least 6 lugs (18), more particularly at least 7 lugs (18), more particularly at least 8 lugs (18), more particularly at least 9 lugs (18), more particularly at least 10 lugs (18), and more particularly at least 12 lugs (18).

9. Arrangement according to any one of the preceding claims,
**characterised in that**
the wire has at least one loose sleeve, which is arranged as an X-ray marker proximally to the second sleeve (14).

10. Arrangement according to any one of the preceding claims,
**characterised in that**
the first sleeve (16) is firmly connected, more particular bonded, with the core (15), more particularly by means of welding, adhesion or soldering, or through plastic deformation, more particularly by means of crimping.

11. Arrangement according to any one of claims 1 to 9,
**characterised in that**
the first sleeve (16) is borne in a rotatable manner about the core (15).

12. Arrangement according to claim 11,
**characterised in that**
at its distal side the first sleeve (16) is axially fixed by a coil (12) and on its proximal side by a ring (30).

13. Arrangement according to any one of claims 11 or 12,
**characterised in that**
the ring (30) is firmly connected, more particular bonded, with the core (15), more particularly by means of welding, adhesion or soldering, or through plastic deformation, more particularly by means of crimping.

14. Arrangement according to any one of claims 11, 12 or 13,
**characterised in that**
the radial extent of the ring (30) is smaller than the radial extent of the first sleeve (16).

## Revendications

1. Agencement comprenant un fil (10) et un implant médical (21) pouvant être comprimé qui est relié de façon séparable au fil (10), sachant que le fil (10) présente un premier manchon (16) comprenant au moins un ergot (18) qui s'étend radialement vers l'extérieur, qui est mis en prise avec l'implant (21) pour la transmission de force dans le sens proximal (RP) et qui forme une première butée pour l'implant, sachant qu'un second manchon (14) est disposé proximal au premier manchon (16) qui forme une seconde butée pour la transmission de force dans le sens distal (RD), sachant qu'un espace libre (17) pour recevoir l'implant (21) est prévu, qui est délimité sur le plan distal par le premier manchon (16) et sur le plan proximal par le second manchon (14),
**caractérisé en ce que**
le fil (10) présente une âme (15) qui présente un diamètre plus petit sur l'extrémité distale que sur une extrémité proximale, et l'implant (21) présente au moins un oeillet (22) avec des marqueurs aux rayons X (23) qui est disposé dans l'espace libre (17) lorsque l'implant (21) est comprimé.

2. Agencement selon la revendication 1,
**caractérisé en ce que**
l'extrémité proximale de l'oeillet (22) présente une forme arrondie (24) et sert de butée sur le second manchon (14) pour la transmission de force dans le sens distal RD.

3. Agencement selon la revendication 1 ou 2,
**caractérisé en ce que**
la forme de l'ergot (18) est adaptée à la forme de l'espace intermédiaire (26) des pattes (25) de l'implant.

4. Agencement selon l'une des revendications précédentes,
**caractérisé en ce que**
les cellules terminales avec marqueurs (27) sont formées plus longtemps que les cellules terminales sans marqueurs (28).

5. Agencement selon l'une des revendications précédentes,
**caractérisé en ce que**
le fil (10) présente un tronçon (20) sur l'extrémité distale (RD), qui dans le sens distal (RD), fait saillie axialement sur le premier manchon (16) pour stabiliser l'implant (21).

6. Agencement selon l'une des revendications précédentes,
**caractérisé en ce que**
le premier manchon (16) présente au moins 2 ergots (18), en particulier au moins 3 ergots (18), en particulier au moins 4 ergots (18), en particulier au moins 5 ergots (18), en particulier au moins 6 ergots (18), en particulier au moins 7 ergots (18), en particulier au moins 8 ergots (18), en particulier au moins 9 ergots (18), en particulier au moins 10 ergots (18) et en particulier au moins 12 ergots (18).

7. Agencement selon l'une des revendications précédentes,
**caractérisé en ce que**
l'implant (21) présente un nombre d'oeillets correspondant au nombre d'ergots (18), en particulier trois oeillets (22) qui sont disposés dans un angle de 120°.

8. Agencement selon l'une des revendications précédentes,
**caractérisé en ce que**
le second manchon (14) présente un nombre d'ergots (18) correspondant au nombre d'ergots (18) du premier manchon (16), en particulier au moins un ergot (18), en particulier au moins 2 ergots (18), en particulier au moins 3 ergots (18), en particulier au moins 4 ergots (18), en particulier au moins 5 ergots (18), en particulier au moins 6 ergots (18), en particulier au moins 7 ergots (18), en particulier au moins 8 ergots (18), en particulier au moins 9 ergots (18), en particulier au moins 10 ergots (18) et en particulier au moins 12 ergots (18).

9. Agencement selon l'une des revendications précédentes,
**caractérisé en ce que**
le fil présente au moins un manchon libre qui est disposé de façon proximale au second manchon (14) en tant que marqueur aux rayons X.

10. Agencement selon l'une des revendications précédentes,
**caractérisé en ce que**
le premier manchon (16) est relié fixement à l'âme (15) en particulier par complémentarité de forme, en particulier par soudage, collage ou brasage, ou par déformation plastique, en particulier par sertissage.

11. Agencement selon l'une des revendications 1 à 9,
**caractérisé en ce que**
le premier manchon (16) est disposé rotatif sur l'âme (15).

12. Agencement selon la revendication 11,
**caractérisé en ce que**
le premier manchon (16) est fixé axialement par une bobine (12) sur son côté distal et par un anneau (30) sur son côté proximal.

13. Agencement selon la revendication 11 ou 12,
**caractérisé en ce que**
l'anneau (30) est relié fixement à l'âme (15) en particulier par complémentarité de forme, en particulier par soudage, collage ou brasage, ou par déformation plastique, en particulier par sertissage.

14. Agencement selon l'une des revendications 11, 12 ou 13,
**caractérisé en ce que**
l'allongement radial de l'anneau (30) est inférieur à l'allongement radial du premier manchon (16).
